# EUROPEAN PATENT SPECIFICATION

(11) **EP 3 189 842 B1**
(45) Date of publication and mention of the grant of the patent: **30.10.2019**
(21) Application number: 15837593.1
(22) Date of filing: 27.08.2015
(51) Int. Cl.: A61K 31/704, A61K 8/60, A61P 17/14, A61Q 7/00

(54) **COMPOSITION FOR PREVENTING HAIR LOSS OR PROMOTING HAIR GROWTH, CONTAINING OLEANOLIC ACID DERIVATIVE AND PHARMACEUTICALLY ACCEPTABLE SALT THEREOF**
ZUSAMMENSETZUNG ZUR VERHINDERUNG DES HAARAUSFALLS ODER ZUR FÖRDERUNG DES HAARWUCHSES MIT OLEANOLSÄUREDERIVAT UND EINEM PHARMAZEUTISCH VERTRÄGLICHEN SALZ DAVON
COMPOSITION POUR PRÉVENIR LA CHUTE DES CHEVEUX OU FAVORISER LA POUSSE DES CHEVEUX, CONTENANT UN DÉRIVÉ D'ACIDE OLÉANOLIQUE ET UN SEL PHARMACEUTIQUEMENT ACCEPTABLE DE CELUI-CI

(30) Priority: 04.09.2014 KR 20140117819; 04.09.2014 KR 20140117829
(43) Date of publication of application: 12.07.2017
(73) Proprietor: College of Medicine Pochon Cha University Industry-Academic Cooperation Foundation, Pocheon-si, Gyeonggi-do 11160 (KR)
(72) Inventor: KANG, Sang-Jin, Yongin-si Gyeonggi-do 16990 (KR); KIM, Young-Ho, Daejeon 34191 (KR); LEE, Sung-Hou, Yongin-si Gyeonggi-do 16874 (KR); LEE, Min-Ho, Daejeon 34073 (KR); LEE, Eun-Young, Seoul 04995 (KR); CHOI, Eun-Ju, Yongin-si Gyeonggi-do 17040 (KR); NOH, Hyo-Jin, Cheonan-si Chungcheongnam-do 31066 (KR); NAM, You-Jin, Gyeonggi-do 13540 (KR)
(74) Representative: Turner, Craig Robert
(86) International application number: PCT/KR2015/008964
(87) International publication number: WO 2016/036051

(56) References cited:
- BR-A2- PI0 905 267
- ES-A1- 2 396 075
- JP-A- S6 293 215
- JP-A- H09 157 139
- JP-A- H10 265 328
- JP-A- 2006 206 468
- US-A- 5 607 693
- US-A1- 2006 067 905
- DATABASE GNPD [Online] MINTEL; May 2014 (2014-05), "The relief shampoo", XP002776739, Database accession no. 2425049
- LI WEI ET AL: "Oleanane-type triterpenoid saponins from the roots ofPulsatilla koreanaand their apoptosis-inducing effects on HL-60 human promyelocytic leukemia cells", ARCHIVES OF PHARMACAL RESEARCH, NATL. FISHERIES UNIVERSITY, PUSAN, KR, vol. 36, no. 6, 2 March 2013 (2013-03-02), pages 768-774, XP035312561, ISSN: 0253-6269, DOI: 10.1007/S12272-013-0042-5 [retrieved on 2013-03-02]
- F DELMAS ET AL: "Antileishmanial Activity of Three Saponins Isolated from Ivy, [alpha]-Hederin, [beta]-Hederin and Hederacolchiside A 1 , as Compared to Their Action on Mammalian Cells Cultured in Vitro", PLANTA MEDICA, vol. 66, no. 4, 29 May 2000 (2000-05-29), pages 343-347, XP055435300, DE ISSN: 0032-0943, DOI: 10.1055/s-2000-8541
- CHEN, LI ET AL.: 'Synthesis and biological evaluation of nitric oxide-releasing derivatives of oleanolic acid as inhibitors of HepG2 cell apoptosis.' BIOORGANIC & MEDICINAL CHEMISTRY LETTERS vol. 17, no. 11, 10 May 2007, pages 2979 - 2982, XP022068376 DOI: 10.1016/J.BMCL.2007.03.068
- MUNDADA, SNEHA ET AL.: 'Pharmacology of Tridax procumbens a Weed: Review.' INTERNATIONAL JOURNAL OF PHARMTECH RESEARCH vol. 2, no. 2, 01 January 2010, pages 1391 - 1394, XP055198747

## Description

### TECHNICAL FIELD

The present invention relates to a composition for preventing hair loss or promoting hair growth, comprising an oleanolic acid derivative or a pharmaceutically acceptable salt thereof as an active ingredient.

### BACKGROUND ART

Corticotropin releasing hormone (CRH) is one of the major components of the hypothalamic-pituitary-adrenal axis (HPA axis) and is activated by mental stress. CRH, a peptide consisting of 41 amino acids, is a major contributor of the HPA axis. In response to stress, CRH plays an important role in the hormone and neurotransmitter cascade in vertebrates.

Serious psycho-emotional stress affects hair growth significantly and causes the development of alopecia areata. It has been reported that CRH inhibits hair shaft elongation and promotes apoptosis of hair matrix keratinocytes in an *ex vivo* culture of human hair follicles (Ito N, et al., Human hair follicles display a functional equivalent of the hypothalamic-pituitary-adrenal(HPA) axis and synthesize cortisol, FASEB J. 19 (2005) 1332-4). In addition, it has been reported that CRH receptor antagonists induce hair growth in CRH-overexpressing hairless mice (Lixin Wang, et al., CRH receptor antagonist astressin-B reverses and prevents alopecia in CRH over-expressing mice, Plos One. 6(2011) e16377-e16385). It has been also reported that cultured human hair follicles respond to CRH stimulation and exhibit the HPA axis, including regulatory feedback systems (Natsuho Ito, et al., Human hair follicles display a functional equivalent of the hypothalamic-pituitary-adrenal (HPA) axis and synthesize cortisol, Faseb J. 19(2005)1332-4).

Accordingly, CRH plays an important role in human hair growth, and especially it is expected that a substance capable of regulating expressions of the cytokines regulated by CRH can be used as a material for preventing hair loss or promoting hair growth.

Database GNPD Online Mint; May 2014 (2014-05), "The relief shampoo" Record ID 2425049 relates to a shampoo composition for giving healthy hair and scalp care comprising various extracts including a Pulsatilla koreana extract.

Li Wei et al: "Oleanane-type triterpenoid saponins from the roots of Pulsatilla Korean and their apoptosis-inducing effects on HL-60 human promyelocytic leukemia cells - Article in Archives of Pharmacal Research 36(Vol. 6) ·March 2013, pages 768-774 is an article in which various components in the Pulsatilla koreana extract is discussed, along with the results obtained by evaluating the apoptosis inducing effects thereof.

ES 2396075 relates to a cosmetic composition for regenerating and/or hydrating the skin comprising various extracts including a Hedera helix colchica extract.

BR PI0 905 267 relates to a cosmetic cream comprising saponins extracted from Hedera helix and Hedera colchica with the application of nanotechnology for the development of cosmetic cream.

US 2006/067905 relates to use of oleanolic acid, preferably oleanolic acid glycosides in the treatment of hair loss based on the finding that oleanolic acid inhibits the transformation of testosterone into dihydrotestosterone.

JP H09 157139 relates to a trichogenous agent containing a triterpene-based compound, preferably oleanlic acid, crategolic acid, celastrol or one of their salts, in an amount of 0.001-10wt.% based on the total amount of the agent, and its use as a hair tonic, a shampoo, a rinse, pomade, hair lotion, hair cream and a hair treatment, etc.

### DISCLOSURE

### Technical Problem

The present inventors have evaluated the effect of CRH on human hair dermal papilla cells which play an important role in hair formation. The present inventors have found that CRH inhibits expressions of the hair growth-related cytokines, namely KGF, TGFβ-2, Wnt5a and Nexin and promotes the transition from anagen phase to catagen phase in the hair cycle in the follicular culture system. In order to identify a substance capable of blocking the action of CRH, including CRH receptor antagonists, the present inventors isolated various compounds from various herbal products and their extracts which have been proven to have safety; and performed extensive screenings for finding out the materials showing antagonistic effects on the CRH receptor. As the results thereof, it has been found that a certain oleanolic acid derivative remarkably inhibits the transition of the hairs in the anagen phase to the hairs in the catagen phase by CRH.

Therefore, it is an object of the present invention to provide a composition for preventing hair loss or promoting hair growth, comprising the oleanolic acid derivative or its salt as an active ingredient

### Technical Solution

In accordance with an aspect of the present invention, there is provided a composition for use in preventing a stress-induced hair loss or promoting hair growth in a subject having a stress-induced hair loss, comprising a compound of Formula 1 or 2 or a pharmaceutically acceptable salt thereof as in an amount enough for preventing the stress-induced hair loss or promoting hair growth in said subject an active ingredient.

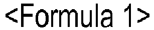

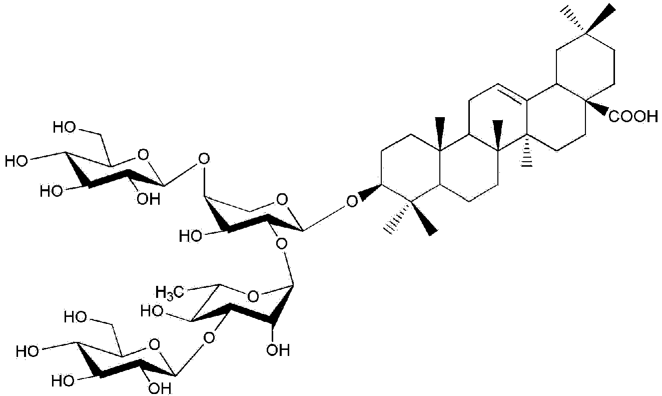

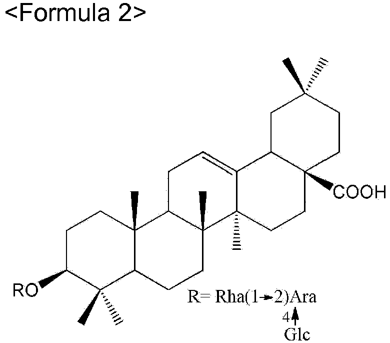

In the composition of the present invention, the hair loss is a stress-induced hair loss.

### ADVANTAGEOUS EFFECTS

It has been found by the present invention that a certain oleanolic acid derivative having the chemical structure of Formula 1 or 2 remarkably inhibits the anagen-catagen transition of hair, which is originated from antagonistic action on the receptor of the stress hormone CRH. Accordingly, the compound of Formula 1 or 2 or a pharmaceutically acceptable salt thereof can be usefully used in a composition for preventing hair loss (particularly for preventing stress-induced hair loss) or promoting hair growth.

### BEST MODE

The present invention provides a composition for use in preventing a stress-induced hair loss or promoting hair growth in a subject having a stress-induced hair loss, comprising a compound of Formula 1 or 2 or a pharmaceutically acceptable salt thereof in an amount enough for preventing the stress-induced hair loss or promoting hair growth in said subject as an active ingredient.

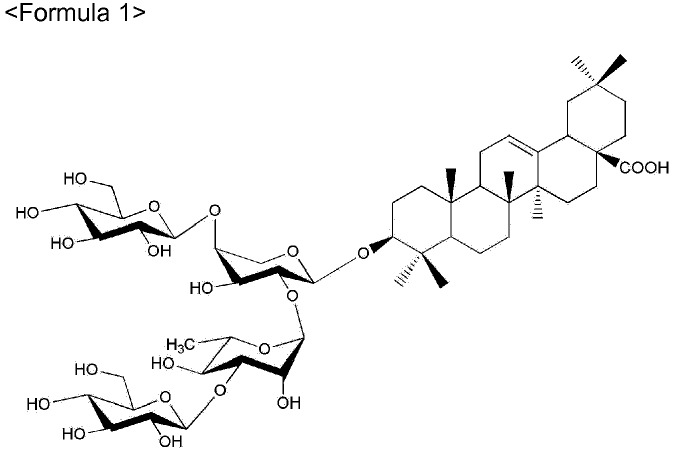

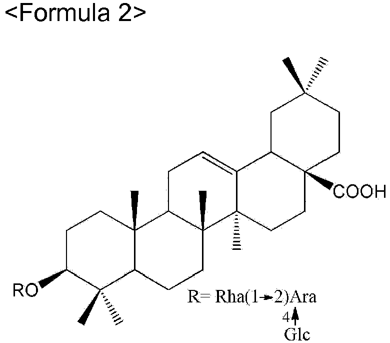

The compound of Formula 1 is a known material and its chemical name is 3-O-β-D-glucopyranosyl (1→3)-α-L-rhamnopyranosyl (1→2) [β-D-glucopyranosyl (1→4)]-α-L-arabinopyranosyl oleanolic acid. The CAS number thereof is 106577-41-7. The compound of Formula 2 is also a known material and its chemical name is oleanolic acid-3-O-α-L-rhamnopyranosyl-(1→2)-[β-D-glucopyranosyl-(1→4)]-α-L-arabinopyranosi de. The CAS number thereof is 106577-39-3. The compound of Formula 2 is also referred to as 'Raddeanoside R13' or 'Hederacolchiside A1'. The compounds of Formulas 1 and 2 can be obtained according to the methods known in the arts, for example, Wei Li et al, Oleanane-type triterpenoid saponins from the roots of Pulsatilla koreana and their apoptosis-inducing effects on HL-60 human promyelocytic leukemia cells, Arch. Pharm. Res. (2013) 36, pp768-774, and the like. The salt of the compound of Formula 1 or 2 includes a pharmaceutically acceptable conventional salt, e.g., an alkali metal salt such as sodium salt, an alkaline earth metal salt such as calcium salt, and the like, but is not limited thereto.

The composition of the present invention can be usefully applied to prevent hair loss. The hair loss includes alopecia such as alopecia areata and diffuse alopecia, according to the hair loss forms, and alopecia such as stress-induced hair loss and female hair loss, according to the hair loss causes. The composition of the present invention can be applied preferably to a stress-induced hair loss among the hair losses originated from various causes.

The composition of the present invention may be formulated in the form of a pharmaceutical composition and/or a cosmetic composition. For example, the composition of the present invention may be in the form of a pharmaceutical composition for preventing hair loss or promoting hair growth, comprising the compound of Formula 1 or 2 or a pharmaceutically acceptable salt thereof as an active ingredient. In addition, the composition of the present invention may be in the form of a cosmetic composition for preventing hair loss or promoting hair growth, comprising the compound of Formula 1 or 2 or a pharmaceutically acceptable salt thereof.

The pharmaceutical composition may include a pharmaceutically acceptable carrier and may be formulated into a parenteral formulation such as a solution, a suspension, an emulsion, a lotion, an ointment or a lyophilized formulation according to conventional methods. Preferably, the pharmaceutical composition may be formulated into a transdermal dosage form such as a solution for external use, an emulsion, and an ointment. The pharmaceutically acceptable carrier includes an aqueous diluent or solvent such as phosphate buffered saline, purified water, sterilized water, etc., and a non-aqueous diluent or solvent such as propylene glycol, polyethylene glycol, olive oil, etc. And also, if necessary, the pharmaceutically acceptable carrier may include wetting agents, flavoring agents, preservatives, and the like. The dose of the compound of Formula 1 or 2 or a pharmaceutically acceptable salt thereof in the pharmaceutical composition varies depending on condition and body weight of a patient, severity of the disease, dosage form, administration route and time, and can be appropriately selected by a skilled person in the art. For example, the compound of Formula 1 or 2 or a pharmaceutically acceptable salt thereof may be administered at a dose of 0.01 to 1000 mg/kg per day, preferably 0.1 to 100 mg/kg per day. The administration may be carried out once or several times a day.

The composition of the present invention may be in the form of a cosmetic composition comprising the compound of Formula 1 or 2 or a pharmaceutically acceptable salt thereof as an active ingredient. The cosmetic composition of the present invention may be prepared in various forms according to conventional methods thereof, using the compound of Formula 1 or 2 or a pharmaceutically acceptable salt thereof. For example, the cosmetic composition of the present invention may be prepared in forms of a cosmetic product, a shampoo, a hair lotion, a hair cream, a hair gel, a scalp essence, a tonic, etc. containing the compound of Formula 1 or 2 or a pharmaceutically acceptable salt thereof, which may be diluted with a cleansing water, an astringent solution, or a moisture solution, for the use thereof. And also, the cosmetic composition may include conventional excipients, such as a stabilizer, a solubilizing agent, vitamin, a pigment, a flavoring agent, which are conventionally used in the field of cosmetic composition. In the cosmetic composition of the present invention, the compound of Formula 1 or 2 or a pharmaceutically acceptable salt thereof may be present in an amount enough to provide the effects for preventing hair loss or promoting hair growth, for example in an amount ranging from 0.001 to 50 wt%, preferably about 1 to 20 wt%, based on the total weight of the composition.

The present invention will be described in further detail with reference to the following examples. These examples are for illustrative purposes only and are not intended to limit the scope of the present invention.

### Example: Evaluation of the effects of the compounds of Formulas 1 and 2 on anagen-catagen transition in a human hair follicle culture test

Normal human scalp tissues (Bundang Cha Hospital) were washed with 70% ethanol for 2 minutes, with phosphate buffered saline (PBS) for 2 minutes, with 5X Anti-anti (Gibco) for 2 minutes, and then with PBS for 2 minutes, to remove any contamination. The hair follicles in the anagen phase were selected and then cultured in William's medium (containing insulin, hydrocortisone, HEPES, Anti-anti) (hereinafter referred to as "the medium", unless otherwise described) for one day. The hair follicles maintaining the anagen phase were used in the experiment.

The hair follicles in the anagen phase were selected and then one hair follicle per well of a 24-well cell culture plate was cultured along with the medium. 20 Hair follicles were used per group. The test was performed with three groups, i.e., a control group, a positive control group, and test groups. The hair follicles of the control group were cultured in the medium for 3 days and 6 days. The hair follicles of the positive control group were cultured in the medium containing CRH (10⁻⁶ M) for 3 days and 6 days. The hair follicles of each test group were respectively pretreated for 2 hours with the medium containing the compound of Formula 1 or 2 in the concentration of 0.5 uM. CRH (10⁻⁶ M) was added to the medium of each test group, which was then for 3 days and 6 days. After culturing each group for 3 days and 6 days, the numbers of anagen hairs and catagen hairs in each group were observed through a stereoscopic microscope. The results thereof are shown in Table 1 below.

**<Table 1>**

| Inhibitory effects of the compound of Formulas 1 and 2 against anagen-catagen transition | | | | |
|---|---|---|---|---|
| | Control group | Positive control group (CRH) | Test group | |
| | | | Compound of Formula 1 | Compound of Formula 2 |
| 3 days | 15 % | 60 % | 25 % | 10 % |
| 6 days | 60 % | 95 % | 35 % | 10 % |

As can be seen from the results in Table 1, in the control group, 15% and 60% of the hairs were converted from the anagen phase to the catagen phase after culturing for 3 days and 6 days, respectively. In the positive control group, 60% and 95% of the hairs were converted to the catagen phase after culturing for 3 days and 6 days, respectively, because of the CRH effects. In the test group treated with the compound of Formula 1, only 25% and 35% of the hairs were converted to the catagen phase after culturing for 3 days and 6 days, respectively. In the test group treated with the compound of Formula 2, only 10% and 10% of the hairs were converted to the catagen phase after culturing for 3 days and 6 days, respectively. From the above results, it can be seen that CRH promotes the transition of the hair in the anagen phase to the hair in the catagen phase, and the compounds of Formulas 1 and 2 effectively block the effect of CRH, thereby maintaining the hairs in the anagen phase well.

## Claims

1. A composition for use in preventing a stress-induced hair loss or promoting hair growth in a subject having a stress-induced hair loss, comprising an compound of Formula 1 or 2 or a pharmaceutically acceptable salt thereof in an amount enough for preventing the stress-induced hair loss or promoting hair growth in said subject as an active ingredient.

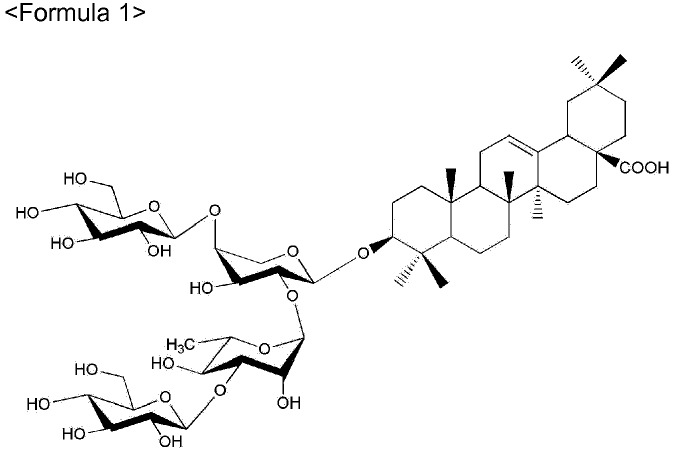

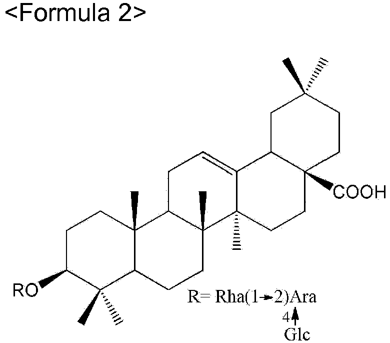

## Patentansprüche

1. Zusammensetzung zur Verwendung bei der Prävention von stressbedingtem Haarausfall oder der Förderung des Haarwuchses bei einer Person mit stressbedingtem Haarausfall, umfassend eine Verbindung nach Formel 1 oder 2 oder ein pharmazeutisch annehmbares Salz davon, in einer zur Prävention des stressbedingten Haarausfalls oder zur Förderung des Haarwuchses bei der Person ausreichenden Menge, als Wirkstoff.

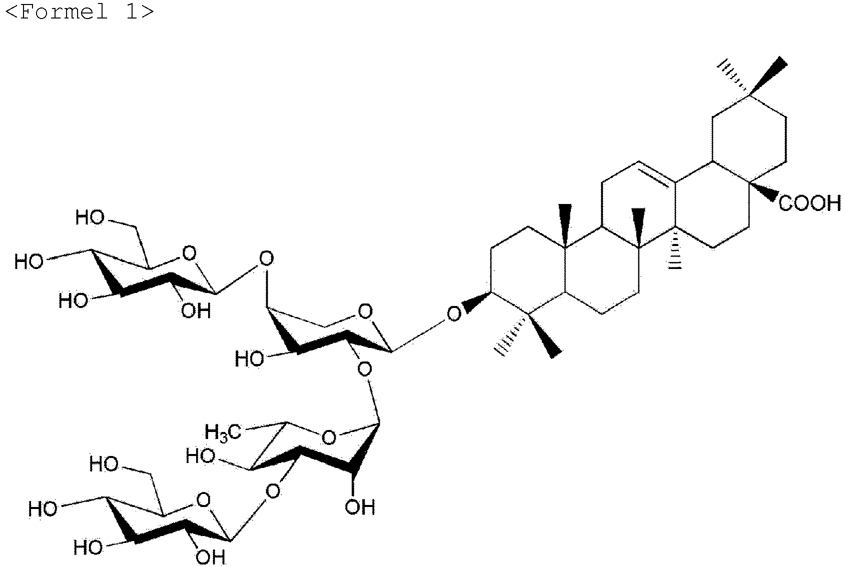

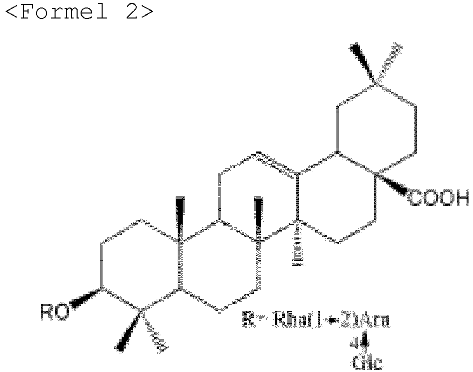

## Revendications

1. Composition destinée à être utilisée dans la prévention de la chute de cheveux induite par le stress ou dans la promotion de la croissance des cheveux chez un sujet souffrant de perte de cheveux induite par le stress, comprenant un composé de la Formule 1 ou 2 ou un de ses sels pharmaceutiquement acceptables en une quantité suffisante pour prévenir la chute de cheveux induite par le stress ou pour promouvoir la croissance des cheveux chez ledit sujet en tant que principe actif.
